# FASCICULE DE BREVET EUROPEEN

(11) **EP 2 797 540 B1**
(45) Date de publication et mention de la délivrance du brevet: **07.10.2020**
(21) Numéro de dépôt: 12794425.4
(22) Date de dépôt: 31.10.2012
(51) Int. Cl.: A61B 34/30, A61B 90/00, A61B 34/20, A61B 17/00

(54) **DISPOSITIF MEDICAL ROBOTISE DE SURVEILLANCE DE LA RESPIRATION D'UN PATIENT ET DE CORRECTION DE LA TRAJECTOIRE D'UN BRAS ROBOTISE**
ROBOTISCHE MEDIZINISCHE VORRICHTUNG ZUR ÜBERWACHUNG DER ATMUNG EINES PATIENTEN UND KORREKTUR DER BAHN EINES ROBOTERARMS
ROBOTIC MEDICAL DEVICE FOR MONITORING THE RESPIRATION OF A PATIENT AND CORRECTING THE TRAJECTORY OF A ROBOTIC ARM

(30) Priorité: 30.12.2011 FR 1162555; 27.04.2012 FR 1253919
(43) Date de publication de la demande: 05.11.2014
(73) Titulaire: Medtech, 34170 Castelnau le Lez (FR)
(72) Inventeur: MAILLET, Pierre, 34130 Saint Aunes (FR); NAHUM, Bertin, 34670 Baillargues (FR); BADANO, Fernand, 69100 Villeurbanne (FR); DEHOUR, Patrick, 30260 Crespian (FR)
(74) Mandataire: Taor, Simon Edward William
(86) Numéro de dépôt international: PCT/FR2012/052532
(87) Numéro de publication internationale: WO 2013/098496

(56) Documents cités:
- US-A1- 2006 089 626
- US-A1- 2006 210 019
- US-A1- 2006 285 632
- US-A1- 2007 185 485
- US-A1- 2008 208 212
- US-A1- 2009 297 005
- US-A1- 2010 063 514

## Description

La présente invention entre dans le domaine médical, en particulier dans la méthodologie opératoire lors de la préparation et la réalisation d'interventions chirurgicales.

L'invention concerne spécifiquement l'imagerie médicale anatomique, dans le but d'effectuer des interventions chirurgicales assistées par la robotique.

L'invention a pour objet un dispositif médical robotisé de surveillance de la respiration d'un patient et de correction de la trajectoire robotisée.

La présente invention trouvera une application préférentielle, mais aucunement limitative, pour des interventions chirurgicales de la zone anatomique du rachis.

On notera que l'invention sera décrite selon un exemple particulier d'intervention au niveau du rachis lombaire ou lombal, au niveau de la courbure antérieure lordose de la colonne vertébrale. Toutefois, l'invention pourra être utilisée par une intervention au niveau des rachis cervicaux supérieur et inférieur, du rachis dorsal ou thoracique, ainsi que du rachis sacré et du coccyx.

Dans ce cadre, un problème majeur lors d'une intervention assistée par robotique réside dans la gestion des mouvements anatomiques du patient consécutifs à sa propre respiration. En particulier, la respiration dépend de l'activité du diaphragme générant les mouvements thoraciques et pulmonaires contributifs aux échanges gazeux. Cette activité musculaire entraine une déformation des parties anatomiques collatérales, comme l'abdomen mais surtout le rachis. L'amplitude de cette déformation dépend de la ventilation minute (VM), en fonction de son volume et de sa fréquence, mais aussi de la position du patient, à savoir débout, assis ou bien allongé sur le ventre, sur le dos ou encore de côté.

Dans le cas d'une intervention sur le rachis, ce dernier se déplace d'une façon plus conséquente pour les vertèbres thoraciques et dans une moindre mesure pour les vertèbres lombaires.

Afin de limiter ces mouvements lors d'une intervention lombaire, par exemple, quand la voie d'accès le permet, le patient est allongé sur le ventre, en prenant la précaution de laisser libre les mouvements du ventre sous le niveau du bassin pectoral. Le patient est alors immobilisé dans cette position par des mécanismes et accessoires de la table d'opération. Ce décubitus ventral particulier permet de diminuer considérablement l'amplitude des mouvements du rachis lombaire,

On pratique ainsi des opérations minutieuses dans la zone du rachis lombaire, comme la laminectomie (canal étroit), la libération radiculaire (hernie discale), l'arthrodèse (fusion de vertèbres avec vissages pédiculaires), la kyphoplastie et la vertébroplastie (injection de ciment dans le corps vertébral).

Néanmoins, la respiration génère des mouvements périodiques du rachis lombaire de quelques millimètres, que le chirurgien est alors obligé de compenser par sa dextérité et son acuité visuelle.

Cette contrainte de compensation est d'autant plus importante avec l'emploi d'un système robotique qui se substituera à la main du neurochirurgien. En effet, la robotique doit garantir une meilleure précision des gestes pour les sécuriser (comme la précision de forage du pédicule, l'identification de l'anatomie en mini invasif ou endoscopie percutanée, ou bien la définition de zones de sécurité pour éviter d'endommager la moelle épinière, veines, nerfs). D'autre part, le robot doit accompagner les mouvements prévisibles de l'anatomie en les anticipant, à l'instar de la main et de l'œil du chirurgien, ceci avec une rapidité adaptée à la vélocité de la cible, autrement les dégâts pourraient être plus conséquents avec le robot qui suit son programme dans un environnement statique.

A l'heure actuelle, une approche simplificatrice consiste à utiliser un télémètre laser ou à ultrasons, dont le faisceau vise, à sa verticale, la peau recouvrant l'épine dorsale d'une vertèbre. On enregistre ainsi l'amplitude du déplacement de ladite vertèbre mais suivant un unique axe vertical. De plus, avec une mesure de surface sur la peau, il n'est pas possible de déterminer avec exactitude si la vertèbre se déplace comme la peau, ni dans quelles directions dans l'espace.

Une autre solution existante consiste à visser un marqueur dans l'épine dorsale du patient vers lequel on dirige l'optique d'un système de mesure tridimensionnelle. Ce système permet de capturer en temps réel les coordonnées dans l'espace dudit marqueur, Ces coordonnées permettent donc de suivre le déplacement dé la vertèbre sur laquelle est positionné le marqueur. Puis, un algorithme calcule informatiquement les compensations dans le repère dans lequel évolue ledit bras robotisé, en corrélation avec l'imagerie médicale tridimensionnelle réalisée préalablement à l'opération.

Toutefois, le déplacement mesuré est limité en précision à la vertèbre sur laquelle est placé le marqueur, des différences pouvant être constatées dans les mouvements des vertèbres les unes par rapport aux autres, ainsi que des organes collatéraux Il serait alors nécessaire de positionner un marqueur sur chaque vertèbre, ou bien au niveau des vertèbres autour de la zone anatomique d'intervention. Rappelons à ce titre que cette solution présente l'inconvénient d'être invasive et que multiplier les marqueurs n'est donc pas une solution satisfaisante dans le cadre de mouvements de plusieurs vertèbres.

Le document US 2006/089626 décrit une méthode pour l'ablation d'une tumeur à l'intérieur du corps du patient. La méthode consiste à positionner la sonde échographique dans une position fixe pendant un cycle respiratoire complet et à capturer des images synchronisées avec le temps d'arrêt. Le temps d'arrêt est la brève période de pause entre l'inhalation et l'expiration à la fin de chaque cycle respiratoire. Les images sont utilisées pour informer un chirurgien lorsque la tumeur est alignée avec le plan d'ablation.

A titre d'exemple, un solution connue est décrite dans le document US 2010/063514. Il s'agit d'un dispositif et une méthode de surveillance des mouvements respiratoires d'un patient, dans le cadre d'opérations médicales invasives au niveau d'une zone anatomique du corps dudit patient.

Pour ce faire, des acquisitions d'images sont effectuées en continu, par l'intermédiaire de rayons X ou bien d'ultrasons. Ensuite, ces images enregistrées permettent de calculer les mouvements de la zone anatomique visée, et d'obtenir une courbe desdits mouvements, de préférence une courbe périodique.

De plus, la surveillance peut faire appel à un respirateur. Dans ce cas, l'horloge interne dudit respirateur peut servir de signal de déclenchement pour l'imagerie pré- ou per-opératoire. Il s'agit simplement d'une utilisation de l'horloge interne du respirateur, en tant que déclencheur. Dès lors, les mouvements ainsi détectés peuvent permettre la correction de trajectoire d'un outil chirurgical en synchronisation avec la respiration du patient, en particulier la trajectoire d'un robot.

Toutefois, ce document ne précise en aucune manière la technique employée, en particulier le calcul effectué, permettant, à partir de la prise d'images successives et en continu, d'obtenir la correction de trajectoire souhaitée.

Le but de la présente invention consiste à pallier les inconvénients de l'état de la technique en proposant un dispositif permettant de simuler les mouvements du rachis lombaire sous l'effet de la respiration, en vue de corriger les mouvements d'un système robotisé, notamment d'un bras robotisé, supportant des outils chirurgicaux et des moyens de traitements actifs (de type laser ou rayonnements à visées thérapeutiques).

L'invention se veut à même de mesurer ces mouvements pour que le bras robotisé s'y adapte automatiquement et puisse même les anticiper, afin de conserver l'amélioration de la précision robotique par rapport à celle du chirurgien, tout en accompagnant lesdits mouvements avec une rapidité d'exécution correspondant la vélocité de la cible.

De plus, l'invention offre une solution présentant l'avantage d'être non invasive.

Pour ce faire, l'invention a pour objet un dispositif médical robotisé de surveillance de la respiration d'un patient et de correction de la trajectoire d'un bras robotisé, comprenant :
- au moins un bras robotisé ;
- un ventilateur mécanique, auquel est soumise la respiration d'un patient ;
- des moyens d'enregistrement en fonction du temps des instants au cours de ladite ventilation mécanique dans lesquels ledit patient se trouve dans une position d'origine correspondant à la position du patient à la fin de l'expiration des gaz jusqu'à la prochaine insufflation des gaz et dans une position haute correspondant à la position maximale à la fin de l'insufflation ;
- des moyens de capture numérique d'images d'une zone anatomique dudit patient, le déclenchement desdits moyens de capture étant synchronisé avec les instants enregistrés dans ladite position d'origine et ladite position haute ;
- des moyens de calcul d'au moins un vecteur de déplacement tridimensionnel de ladite zone entre lesdites positions d'origine et haute ;
- des moyens de correction de la trajectoire dudit bras robotisé en fonction de chaque vecteur tridimensionnel calculé.

Une telle solution repose tout d'abord sur le fait que les interventions sur le rachis sont généralement effectuées sous anesthésie dite « générale ». Dès lors, la ventilation du patient est réalisée par un appareil respiratoire assurant une ventilation mécanique. Dans ces conditions, il est alors possible de connaître de façon précise et répétitive les paramètres de la respiration du patient anesthésié.

Les moyens mis en œuvre dans l'invention prennent en compte ces paramètres et les interprètent afin de compenser le déplacement du bras robotisé en fonction des mouvements respiratoires du patient.

En particulier, l'invention prévoit précisément d'enregistrer deux positions distinctes à deux instants temporels différents. Ces positions sont choisies parmi toutes les positions possibles lors du mouvement du corps du patient. Il s'agit en fait des positions extrêmes de déplacement de la zone anatomique, qui coïncident avec l'insufflation et l'expiration automatique générée par le ventilateur artificiel.

De plus, l'invention intègre des moyens permettant de détecter les instants dans lequel le patient se retrouve dans ces positions, en utilisant les paramètres de fonctionnement dudit respirateur. Ces mêmes moyens permettent alors de contrôler le déclenchement à ces instants précis, sans se servir de l'horloge interne dudit respirateur comme d'un déclencheur. En somme, le déclenchement de la capture d'images s'effectue de façon synchrone grâce à l'enregistrement préalablement réalisé. Ainsi, l'invention met en œuvre une synchronisation temporelle différente du simple calage sur l'horloge interne du respirateur.

En outre, l'enregistrement s'effectue de façon discontinue, par des clichés différents et pris à des instants distincts dans le temps, de manière à ne pas soumettre outre mesure le patient à des ondes et des radiations.

Par ailleurs, le dispositif selon l'invention fait intervenir des moyens de calcul d'un vecteur de déplacement entre les deux images prises aux deux instants. Ce vecteur sert à la correction de la trajectoire du bras robotisé, au travers de moyens adaptés pour transmettre cette correction audit bras.

Selon d'autres caractéristiques, un tel dispositif se caractérise en ce que lesdits moyens de captures comprennent, des capteurs à ultrasons, ces derniers étant positionnés au contact de ladite zone anatomique.

On notera que cette mesure par ultrasons peut consister en une échographie.

Avantageusement, lesdits moyens de capture comprennent un fluoroscope.

De plus, ledit dispositif comprend des moyens de superposition des clichés capturés par ledit fluoroscope et en ce que lesdits moyens de calcul comprennent, d'une part, un module de détermination d'au moins un vecteur bidimensionnel de déplacement de ladite zone anatomique à partir des clichés superposés et, d'autre part, un module de recoupement desdits vecteurs bidimensionnels pour obtenir ledit vecteur tridimensionnel.

De préférence, lesdits moyens de superposition comprennent des moyens de traitement informatique desdites images capturées par segmentation du contour d'au moins un élément anatomique de ladite zone anatomique apparaissant sur chaque image et en ce que lesdits moyens de superposition consiste à superposer lesdits contours ainsi traités.

En particulier, lesdits moyens de traitement informatique comprennent une interface de pointage manuel.

En outre, lesdits moyens de correction de la trajectoire du bras robotisé comprennent un module informatique de recalage desdites images anatomiques capturées par mise en correspondance avec une imagerie médicale capturée préalablement, ledit recalage étant effectué avec des imageries réalisées de préférence en ladite position d'origine.

On notera qu'il est possible d'utiliser des moyens techniques alternatifs de capture ou de mesure anatomique afin d'obtenir un résultat équivalent.

D'autres caractéristiques et avantages de l'invention ressortiront de la description détaillée qui va suivre des modes de réalisation non limitatifs de l'invention, en référence aux dessins annexés, à savoir :
- les figures 1A et 1B représentant deux exemples de clichés fluoroscopiques correspondant à des captures anatomiques au moment où le patient se trouve dans une position donnée, lesdits deux clichés étant réciproquement capturés dans le plan latéral et dans le plan antérieur / postérieur ;
- les figures 2A et 2B représentant deux exemples de l'étape de traitement de segmentation réciproquement d'un premier cliché latéral et d'un second cliché antérieur ; et
- la figure 3 représentant un exemple de superposition des contours de deux segmentations dans le plan latéral entre une position d'origine et une position haute.

La présente invention concerne un dispositif médical robotisé de surveillance de la respiration d'un patient et de correction de la trajectoire d'un bras robotisé.

Dans le cadre d'une telle intervention, le patient est anesthésié, La curarisation affecte le fonctionnement musculaire, dont l'activité du diaphragme, jusqu'à la paralysie. Est alors mise en œuvre une ventilation mécanique qui assure la ventilation du patient. Dès lors, le dispositif comprend un ventilateur mécanique, auquel est soumise la respiration d'un patient. Ainsi, le patient est totalement dépendant de la machine qui lui insufflera toujours le même volume d'air dans un timing bien précis et reproductible indéfiniment, sous réserve d'une physiologie pulmonaire stable.

Dans ce contexte, les mouvements thoraciques, abdominaux, dorsaux, selon la position du patient, et consécutifs à la ventilation mécanique seront toujours reproductibles et donc prévisibles. Pour une anesthésie générale, la ventilation mécanique s'effectue généralement en Volume Contrôlé, on peut employer aussi la Pression Contrôlée.

Dans un mode de fonctionnement d'une ventilation en mode volumétrique, le volume est une consigne qui sera caractérisée par un débit constant durant un temps d'insufflation constant.

Généralement, le cycle ventilatoire est ajusté selon plusieurs paramètres : le temps ou la durée d'insufflation Tᵢ ajusté à un tiers du cycle, et le temps ou la durée d'expiration Tₑ ajusté à deux tiers du cycle. L'expiration des gaz par le patient correspond, au moins, à la moitié du temps d'expiration alloué et est invariable.

Ainsi, durant le temps d'insufflation T₁, les poumons du patient se gonflent, entrainant une déformation des parties anatomiques collatérales, jusqu'à une limite qui sera atteinte à la fin de la durée inspiratoire. A l'inverse, durant l'expiration du patient, ses poumons se dégonflent d'eux-mêmes du fait de leur propriété élastique, entraînant une déformation des parties anatomiques collatérales, pour finalement reprendre leur position d'origine au terme de l'expiration des gaz, dans un temps inférieur à la durée d'expiration Tₑ allouée.

Entre la fin d'expiration des gaz du patient, et jusqu'à la prochaine insufflation, le patient est théoriquement immobile dans une position dite « d'origine ».

A titre d'exemple, pour un adulte, une fréquence ventilatoire de quinze cycles par minute, donne une durée totale de quatre secondes par cycle, pour une seconde d'insufflation, et un temps d'expiration alloué de trois secondes. L'expiration des gaz sera d'environ deux secondes. Ainsi, la durée d'immobilité sera d'une seconde.

On notera que des ajustements substantiels de la fréquence respiratoire F, sous forme d'une diminution ou d'une augmentation du temps de cycle ventilatoire, tout en conservant la même durée d'insufflation, peuvent augmenter sensiblement le temps d'immobilisation du patient, moyennant une qualité de ventilation satisfaisante durant un temps déterminé et jugé acceptable par l'anesthésiste. On peut de même envisager pour augmenter le temps d'immobilisation, une diminution du temps d'insufflation Tᵢ, tout en augmentant le débit en conséquence afin de garder la même consigne de volume courant, ceci à fréquence respiratoire constante.

Tout d'abord, selon une première caractéristique essentielle, lors du fonctionnement du dispositif selon l'invention, est effectuée une surveillance de la ventilation du patient de manière à déterminer quand le patient est immobile dans sa position d'origine, quand la déformation commence, quand elle atteint son maximum dans une position dite « haute » et quand le mouvement respiratoire se termine.

Pour ce faire, les ventilateurs mécaniques pulmonaires utilisent généralement des capteurs de débit et pression, une horloge interne, pour surveiller lesdits paramètres, à savoir le temps d'insufflation Tᵢ des gaz, le temps d'expiration des gaz, le temps d'expiration alloué Tₑ. Ainsi, des capteurs placés dans le circuit du système de ventilation mécanique permettent d'effectuer en temps réel et en continu les mesures desdits paramètres. Il est alors possible par un traitement approprié de savoir, quand le patient est immobile, quand le mouvement de la cage thoracique commence, quand le mouvement atteint sa plus grande amplitude, quand la cage thoracique retrouve sa position d'origine, comme toutes autres parties anatomiques collatérales. Ce traitement est effectué par des moyens d'enregistrement, en fonction du temps, des instants au cours de ladite ventilation mécanique dans lesquels ledit patient se trouve dans ces positions précises et bien déterminées d'origine et haute.

Dès lors, l'invention enregistre l'instant tₒ au moment où le patient se trouve dans sa position d'origine et l'instant tₕ au moment où le patient se trouve dans sa position haute.

Plus précisément, lesdits moyens enregistrent une courbe périodique, qui permettra de déterminer la position de repos ou d'origine, puis la position haute du patient dans le temps; sachant que les paramètres Tᵢ, Tₑ et F du ventilateur pulmonaire sont parfaitement connus et invariables.

A titre d'exemple aucunement limitatif, lors d'une intervention, selon les pratiques usuellement constatées, pour une fréquence de douze cycles de 5 secondes chacun, on mesure un T₁ de 1,7 secondes et un Tₑ de 3,3 secondes, avec un période d'immobilité de 0,3 seconde.

A partir de ces données temporelles, l'invention prévoit avantageusement de mesurer dans l'espace le positionnement anatomique lorsque le patient se trouve dans les deux positions d'origine et haute, sans chercher à mesurer avec exactitude l'amplitude ni le vecteur de déformation de la colonne vertébrale, ou bien d'une vertèbre lombaire en particulier.

Pour ce faire, le dispositif selon l'invention comprend des moyens de capture numérique d'images anatomiques dudit patient, le déclenchement desdits moyens de capture étant réalisé en fonction de ladite courbe périodique. En particulier, le déclenchement desdits moyens de capture est synchronisé avec les instants enregistrés dans ladite position d'origine et ladite position haute. En d'autres termes, ces captures interviennent au moins à l'instant tₒ au moment où le patient se trouve dans sa position d'origine et au moins à l'instant tₕ au moment où le patient se trouve dans sa position haute.

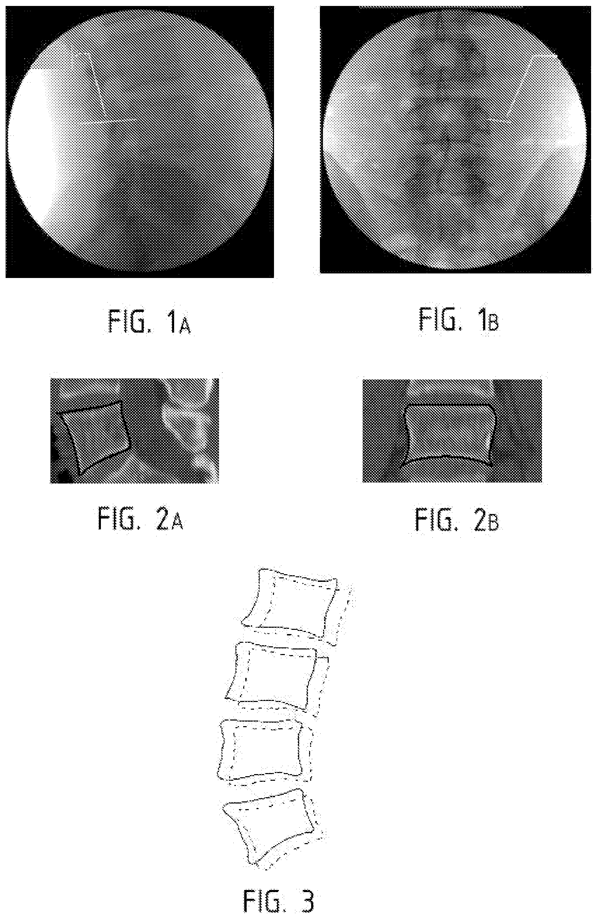

Selon le mode préférentiel de réalisation, ces étapes de capture font appel à une technologie fluoroscopique. Ainsi, chaque capture s'effectue par la prise d'au moins un cliché au moyen d'un fluoroscope : comme par exemple un cliché dans le plan latéral (de côté ou de profil), le plus représentatif du déplacement du rachis pendant la respiration. Un second cliché dans le plan antérieur / postérieur (de face) permet de prendre en compte des mouvements horizontaux, comme visible sur les deux figures 1A et 1B.
On notera que d'autres angles de prise de vue peuvent être envisagés où l'angle de vue du plan de chaque cliché peut être modifié en fonction de la position du patient ou des éléments anatomiques à capturer.

En somme, dans la mise en œuvre du dispositif selon l'invention, cette étape consiste, une fois le patient anesthésié, immobilisé et en position sur la table d'opération (en décubitus ventral), puis l'enregistrement de la courbe périodique de la ventilation préalablement effectué, à déclencher le fluoroscope pour deux premiers clichés, latéral et antérieur de préférence, en se synchronisant sur la position d'origine du patient. Puis, il déclenchera deux seconds clichés en se synchronisant sur la position haute.

Selon un autre mode de fonctionnement, alternatif ou complémentaire, ces étapes de capture peuvent être réalisées par ultrasons. Le dispositif comprend alors des capteurs ultrasons positionnés au contact de la zone anatomique du patient, en vis-à-vis de la vertèbre concernée par l'acte, avec une force d'application asservie et appropriée grâce à un capteur d'effort. Les mesures par ultrasons permettent de capturer lesdites positions de la vertèbre.

On notera que la collecte par ultrasons des mesures des points de la vertèbre par un ou plusieurs capteurs ultrasons, s'effectue lors de l'immobilité du patient, à savoir dans la position d'origine en fin d'expiration des gaz du patient ; puis en synchronisation sur la position haute. De plus, à partir des mesures en positions d'origine et haute, il est possible d'extrapoler avec exactitude les positions intermédiaires, sans générer d'erreur significative vis-à-vis de la précision de registration et du système robotique. Une telle extrapolation permet de s'affranchir d'une multiplication des mesures par ultrason, toutefois possible.

A partir des première et seconde paires de clichés de fluoroscopie ainsi obtenus, un traitement informatique est réalisé.

Ce traitement consiste tout d'abord à segmenter sur chaque cliché le contour en deux dimensions d'un élément anatomique dédié, dans le présent exemple visible sur les figures 2A et 2B montrant le corps vertébral de la vertèbre visée. Il est aussi possible de segmenter plusieurs éléments anatomiques; en particulier les contours de plusieurs vertèbres.

A partir des contours ainsi obtenus, il est possible pour chaque paire de clichés, latéraux et antérieurs de préférence, d'effectuer une superposition des contours selon les deux positions d'origine et haute. L'image suivante montre une superposition des contours des deux positions dans le plan latéral, comme visible sur la figure 3, montrant en trait continu la position d'origine et en trait pointillé la position haute.

Pour ce faire, ledit dispositif comprend des moyens de superposition des clichés capturés par ledit fluoroscope et lesdits moyens de calcul comprennent, d'une part, un module de détermination d'au moins un vecteur bidimensionnel de déplacement de ladite zone anatomique à partir des clichés superposés et, d'autre part, un module de recoupement desdits vecteurs bidimensionnels pour obtenir ledit vecteur tridimensionnel.

Plus précisément, lesdits moyens de superposition comprennent des moyens de traitement informatique desdites images capturées, notamment pour segmenter le contour d'au moins un élément anatomique de ladite zone anatomique apparaissant sur chaque image et lesdits moyens de superposition consistent à superposer lesdits contours ainsi traités.

De plus, lesdits moyens de traitement informatique peuvent comprendre une interface de pointage manuel, permettant au praticien de définir des points spécifique de la zone anatomique, en particulier des points du contour, par exemple d'une vertèbre, afin de guider la segmentation automatique effectuée.

A partir de ces superpositions, une par paire de clichés latéraux et/ou antérieurs de préférence, le dispositif permet de déterminer un vecteur en deux dimensions de déplacement pour chacune des vertèbres selon un plan respectivement latéral (vertical ou sensiblement vertical) et antérieur (horizontal ou sensiblement horizontal).

De plus, à partir de ces vecteurs bidimensionnels latéral et antérieur, leurs recoupements permettent d'obtenir un vecteur tridimensionnel de déplacement pour chaque vertèbre. Ainsi, le dispositif comprend des moyens de calcul d'au moins un vecteur de déplacement tridimensionnel de ladite zone entre lesdites positions d'origine et haute.

Ensuite, à partir de ces vecteurs 3D, l'invention comprend une étape de construction d'une simulation du déplacement temporel et tridimensionnel de chaque vertèbre, coordonnée avec les paramètres F, Tᵢ et Tₑ donnés par le ventilateur pulmonaire. Une telle simulation peut notamment consister en au moins une courbe représentant le déplacement d'un ou plusieurs points de chaque vertèbre.

De plus, à partir des contours en positions d'origine et haute, il est possible d'extrapoler avec exactitude les positions intermédiaires, sans générer d'erreur significative vis-à-vis de la précision de registration et du système robotique. Une telle extrapolation permet de s'affranchir d'une multiplication des clichés fluoroscopique, toutefois possible mais qui soumet le patient et le personnel à une exposition accrue et néfaste aux rayons X.

On notera que les quatre clichés latéral d'origine, latéral haut, antérieur d'origine et antérieur haut, choisis pour l'exemple, sont établis dans un repère connu identique, celui du fluoroscope, identique à celui de système robotisé. Pour ce faire, la mire de repérage dudit fluoroscope peut être directement portée par un bras robotisé.

Dès lors, il est possible, à partir de la simulation de déplacement tridimensionnelle obtenue, de corriger et modifier, même de manière anticipée, la trajectoire d'intervention du système robotisé. Ainsi, le dispositif comprend des moyens de correction de la trajectoire dudit bras robotisé en fonction de chaque vecteur tridimensionnel calculé.

Plus précisément, lesdits moyens de correction de la trajectoire du bras robotisé comprennent un module informatique de recalage desdites images anatomiques capturées par mise en correspondance avec une imagerie médicale capturée préalablement. En effet, les systèmes robotisés existants utilisent une mise en correspondance ou registration d'imagerie médicale en trois dimensions, provenant notamment d'un scanner ou d'un IRM pour « Imagerie à Résonance Magnétique », et d'imagerie en deux dimensions, comme la fluoroscopie.

Tout d'abord, l'imagerie 3D réalisée en préopératoire permet de réaliser une planification de l'opération, en particulier des gestes opératoires et des trajectoires qui seront suivies par chaque bras robotisé. D'autre part, l'imagerie 2D en per-opératoire permet de réaliser des clichés de contrôle en vue de s'assurer du positionnement de l'anatomie du patient, comme évoqué précédemment, et de répercuter cette position aux instruments chirurgicaux, mais aussi de connaitre leurs positions dans un repère 3D du navigateur ou du système robotique. En somme, la registration des images 3D et 2D est un moyen de naviguer l'outil dans l'imagerie 3D préopératoire et vice versa.

Dans ce contexte, des précautions sont prises durant un scanner pour réaliser une imagerie où les mouvements ou la respiration du patient ne viendront pas dégrader le résultat. On demande alors au patient de ne pas bouger, et certains systèmes sont capables de corriger les mouvements de la respiration. L'imagerie 3D est donc considérée comme le résultat d'un patient immobile avec une respiration bloquée. Cette immobilité est d'autant plus vraie pour la partie basse du rachis, les lombaires, lorsque le patient est allongé sur le dos. L'imagerie 3D préopératoire sert donc de référence.

Comme évoqué précédemment, lorsque le patient est placé en condition chirurgicale (decubitus ventral), la position d'une vertèbre dépendra du mouvement respiratoire. Dans le cas de la fluoroscopie 2D en per-opératoire, cette dépendance se poursuit logiquement. Ainsi, le système d'imagerie fluoroscopique 2D de contrôle per-opératoire sert de lien pour rassembler les imageries et les robots dans le même repère. Il est également sous entendu que l'opération de recalage du bras robot, via la mire de repérage, dans le repère de l'image fluoroscopique se fera lors de l'immobilité du patient à savoir en position d'origine.

Dès lors, quand il s'agit de mettre en correspondance l'imagerie préopératoire 3D et la fluoroscopie 2D, l'invention prévoit d'utiliser les clichés latéral et antérieur de préférence, en position d'origine du patient. Cette mise en correspondance peut s'effectuer au travers de logiciels connus, notamment de recalage surfacique.

A partir de cet étalonnage préalable des clichés de fluoroscopie 2D, par la reconnaissance et la mise en correspondance de la position d'origine durant le temps d'immobilité totale du patient, l'invention corrige ensuite en temps réel le repère de la registration d'origine à l'aide de ladite simulation des déplacements vertébraux, ceci durant l'opération chirurgical stéréotaxique, qui peut elle aussi être réalisée par un robot également.

Cette fonctionnalité permet de s'assurer de l'immobilité de la région corporelle d'intérêt, définie comme la position d'origine durant l'opération de registration. De plus, elle permet de corriger le recalage en temps réel et consécutif aux mouvements respiratoires du patient.

Ainsi, le dispositif médical robotisé de surveillance de la respiration d'un patient et de correction de la trajectoire robotisée selon la présente invention assure, au travers d'une analyse en temps réel de la ventilation mécanique et automatique d'un patient, la mesure de la position d'origine d'immobilité anatomique et de la mesure d'une position haute de déformation maximale, une simulation de la zone anatomique d'intérêt, en particulier les vertèbres, qui se veut reproductible au cours des cycles respiratoires se succédant lors d'une intervention chirurgicale. Une telle simulation permet alors de corriger dans l'espace et le temps le repère de travail du robot sur la zone d'intérêt anatomique, avec une précision spatiale submillimétrique et une précision temporelle d'une trentaine de millisecondes.

On notera que la description de la présente invention est appliquée à titre d'exemple au rachis lombaire, mais peut s'étendre à toute autre partie adéquate de l'anatomie humaine du rachis.

## Revendications

1. Dispositif médical robotisé de surveillance de la respiration d'un patient et de correction de la trajectoire robotisée, comprenant ;
- au moins un bras robotisé ;
- un ventilateur mécanique, auquel est soumise la respiration d'un patient ;
- des moyens d'enregistrement en fonction du temps des instants au cours de ladite ventilation mécanique dans lesquels ledit patient se trouve dans une position d'origine correspondant à la position du patient à la fin de l'expiration des gaz jusqu' à la prochaine insufflation des gaz et dans une position haute correspondant à la position maximale à la fin de l'insufflation ;
- des moyens de capture numérique d'images d'une zone anatomique dudit patient, le déclenchement desdits moyens de capture étant synchronisé avec les instants enregistrés dans ladite position d'origine et ladite position haute ;
- des moyens de calcul d'au moins un vecteur de déplacement tridimensionnel de ladite zone entre lesdites positions d'origine et haut ;
- des moyens de correction de la trajectoire dudit bras robotisé en fonction de chaque vecteur tridimensionnel calculé.

2. Dispositif selon la revendication 1, **caractérisé en ce que** lesdits moyens de captures comprennent, des capteurs à ultrasons, ces derniers étant positionnés au contact de ladite zone anatomique.

3. Dispositif selon l'une quelconque des revendications précédentes, **caractérisé en ce que** lesdits moyens de capture comprennent un fluoroscope.

4. Dispositif selon la revendication 3, **caractérisé en ce qu'**il comprend des moyens de superposition des clichés capturés par ledit fluoroscope et **en ce que** lesdits moyens de calcul comprennent, d'une part, un module de détermination d'au moins un vecteur bidimensionnel de déplacement de ladite zone anatomique à partir des clichés superposés et, d'autre part, un module de recoupement desdits vecteurs bidimensionnels pour obtenir ledit vecteur tridimensionnel.

5. Dispositif selon la revendication 4, **caractérisé en ce que** lesdits moyens de superposition comprennent des moyens de traitement informatique desdites images capturées par segmentation du contour d'au moins un élément anatomique de ladite zone anatomique apparaissant sur chaque image et **en ce que** lesdits moyens de superposition consiste à superposer lesdits contours ainsi traités.

6. Dispositif selon la revendication 5, **caractérisé en ce que** lesdits moyen de traitement informatique comprennent une interface de pointage manuel.

7. Dispositif selon l'une quelconque des revendications précédentes, **caractérisé en ce que** lesdits moyens de correction de la trajectoire du bras robotisé comprennent un module informatique de recalage desdites images anatomiques capturées par mise en correspondance avec une imagerie médicale capturée préalablement, ledit recalage étant effectué avec des imageries réalisées de préférence en ladite position d'origine.

## Patentansprüche

1. Robotisierte medizinische Vorrichtung zur Überwachung der Atmung eines Patienten und zur Korrektur der robotisierten Bahn, umfassend:
- zumindest einen robotisierten Arm;
- ein mechanisches Beatmungsgerät, dem die Atmung eines Patienten unterliegt;
- Aufzeichnungsmittel in Abhängigkeit der Zeit von Zeitpunkten im Verlauf der mechanischen Beatmung, in denen sich der Patient in einer Ursprungsposition befindet, die der Position des Patienten am Ende der Ausatmung von Gasen bis zur nächsten Insufflation von Gasen entspricht, und in einer hohen Position entsprechend der maximalen Position am Ende der Insufflation;
- Mittel zur digitalen Erfassung von Bildern eines anatomischen Bereichs des Patienten, wobei die Auslösung der Mittel zur Erfassung mit den in der Ursprungsposition und der hohen Position registrierten Zeitpunkten synchronisiert ist;
- Mittel zur Berechnung von zumindest einem Vektor für dreidimensionale Verschiebung des Bereichs zwischen der Ursprungsposition und der hohen Position;
- Mittel zur Korrektur der Bahn des robotisierten Arms in Abhängigkeit jedes berechneten dreidimensionalen Vektors.

2. Vorrichtung nach Anspruch 1, **dadurch gekennzeichnet, dass** die Mittel zur Erfassung Ultraschallsensoren umfassen, die in Kontakt mit dem anatomischen Bereich positioniert sind.

3. Vorrichtung nach einem der vorhergehenden Ansprüche, **dadurch gekennzeichnet, dass** die Mittel zur Erfassung ein Fluoroskop umfassen.

4. Vorrichtung nach Anspruch 3, **dadurch gekennzeichnet, dass** sie Mittel zur Überlagerung von durch das Fluoroskop erfassten Aufnahmen umfasst und dass die Mittel zur Berechnung einerseits ein Modul zur Bestimmung von zumindest einem zweidimensionalen Vektor zur Verschiebung des anatomischen Bereichs ab den überlagerten Aufnahmen und andererseits ein Modul zur Überschneidung der zweidimensionalen Vektoren umfassen, um den dreidimensionalen Vektor zu erhalten.

5. Vorrichtung nach Anspruch 4, **dadurch gekennzeichnet, dass** die Mittel zur Überlagerung Mittel zur elektronischen Verarbeitung der Bilder umfassen, die durch Segmentierung der Kontur von zumindest einem anatomischen Element des anatomischen Bereichs erfasst werden, der auf jedem Bild erscheint, und dass die Mittel zur Überlagerung daraus bestehen, die so behandelten Konturen zu überlagern.

6. Vorrichtung nach Anspruch 5, **dadurch gekennzeichnet, dass** die Mittel zur elektronischen Verarbeitung eine Schnittstelle zur manuellen Ausrichtung umfassen.

7. Vorrichtung nach einem der vorhergehenden Ansprüche, **dadurch gekennzeichnet, dass** die Mittel zur Korrektur der Bahn des robotisierten Arms ein Informatikmodul zur Neueinstellung der erfassten anatomischen Bilder durch Zuordnung zu einer zuvor erfassten medizinischen Abbildung umfassen, wobei die Neueinstellung mit Abbildungen erfolgt, die bevorzugt in der Ursprungsposition umgesetzt sind.

## Claims

1. Robotic medical device for monitoring the breathing of a patient and correcting the robotic trajectory, comprising:
- at least one robotic arm;
- a mechanical ventilator to which the breathing of a patient is subjected;
- means for recording as a function of time moments during said mechanical ventilation at which said patient is in an original position corresponding to the position of the patient following the expiration of gases until the next insufflation of gases and in a high position corresponding to the maximum position following insufflation;
- digital image capturing means of an anatomical zone of said patient, the actuation of said capturing means being synchronized with the moments recorded in said original position and in said high position;
- calculating means of at least one three-dimensional movement vector of said zone between said original and high positions;
- correcting means of the trajectory of said robotic arm as a function of each calculated three-dimensional vector.

2. Device according to Claim 1, **characterized in that** said capturing means comprise ultrasound sensors, said sensors being positioned in contact with said anatomical zone.

3. Device according to any one of the preceding claims, **characterized in that** said capturing means comprise a fluoroscope.

4. Device according to Claim 3, **characterized in that** it comprises means for superimposing pictures captured by said fluoroscope and **in that** said calculating means comprise, on the one hand, a determination module of at least one two-dimensional movement vector of said anatomical zone from superposed pictures and, on the other hand, a cross-referencing module of said two-dimensional vectors to obtain said three-dimensional vector.

5. Device according to Claim 4, **characterized in that** said superposition means comprise computer processing means of said images captured by segmentation of the contour of at least one anatomical element of said anatomical zone appearing in each image and **in that** said superposition means involve superposing said contours processed in this manner.

6. Device according to Claim 5, **characterized in that** said computer processing means comprise a manual plotting interface.

7. Device according to any of the preceding claims, **characterized in that** said correcting means of the trajectory of the robotic arm comprise a computer processing module for resetting said captured anatomical images by matching them with previously captured medical images, said resetting being carried out using images preferably taken in said original position.
